# EUROPÄISCHE PATENTSCHRIFT

(11) **EP 2 406 270 B1**
(45) Veröffentlichungstag und Bekanntmachung des Hinweises auf die Patenterteilung: **17.04.2013**
(21) Anmeldenummer: 09763977.7
(22) Anmeldetag: 04.12.2009
(51) Int. Cl.: C07H 5/02, C07H 15/04

(54) **VERFAHREN ZUR HERSTELLUNG VON 4-FLUORO-4-DESOXY-ALPHA-D-GLUCOPYRANOSIDEN**
METHOD FOR MANUFACTURING 4-FLUORO-4-DESOXY-ALPHA-D-GLUCOPYRANOSIDES
PROCÉDÉ DESTINÉ À LA FABRICATION DE 4-FLUORO-4-DESOXY-ALPHA-D-GLUCOPYRANOSIDES

(30) Priorität: 08.12.2008 EP 08291152
(43) Veröffentlichungstag der Anmeldung: 18.01.2012
(73) Patentinhaber: SANOFI, 75008 Paris (FR)
(72) Erfinder: PODESCHWA, Michael, 65926 Frankfurt am Main (DE); ROSSEN, Kai, 65926 Frankfurt am Main (DE); WEHLAN, Hermut, 65926 Frankfurt am Main (DE)
(74) Vertreter: Löwrick, Oliver
(86) Internationale Anmeldenummer: PCT/EP2009/066417
(87) Internationale Veröffentlichungsnummer: WO 2010/066643

(56) Entgegenhaltungen:
- WO-A-2004/052902
- WO-A-2004/052903
- Y. ITTAH, ET AL.: "Preparation of two methyl deoxyfluoro-ß-D-galactopyranosides, and their interaction with galactan-specific immunoglobulin A J539 (Fab')" CARBOHYDRATE RESEARCH, Bd. 95, 1981, Seiten 189-194, XP002523520
- J. E. NAM SHIN, ET AL.: "Specificity of alpha- and beta--D-galactosidase towards analogs of D-glactopyranosides modified at C-4 or C-5" CARBOHYDRATE RESEARCH, Bd. 84, 1980, Seiten 328-335, XP002523521

## Beschreibung

Die vorliegende Erfindung betrifft ein Verfahren zur Herstellung von 4-Fluoro-4-desoxy-α-D-glucopyranosiden allgemeinen Formel (I) 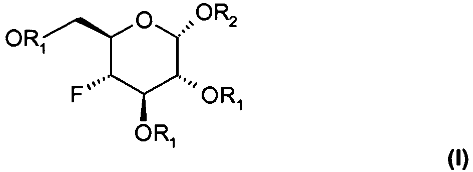 4-Fluoro-4-desoxy- α -D-glucopyranoside sind wichtige Bausteine der Synthese von 4-Fluoro-4-desoxy- α -D-glucopyranosid-glycosiden, welche als Inhibitoren des SGLT1 und/oder SGLT2 eine Anwendung insbesondere in der Prävention und Behandlung von Diabetes Typ 1 und 2 ermöglichen.

W02004/052902 und W02004/052903 beschreiben unter anderem 4-Fluoro-4-desoxy- α -D-glucopyranosid-glycoside und auch ein Verfahren zur deren Herstellung über ein 4-Fluoro-4-desoxy- α -D-glucopyranosid.

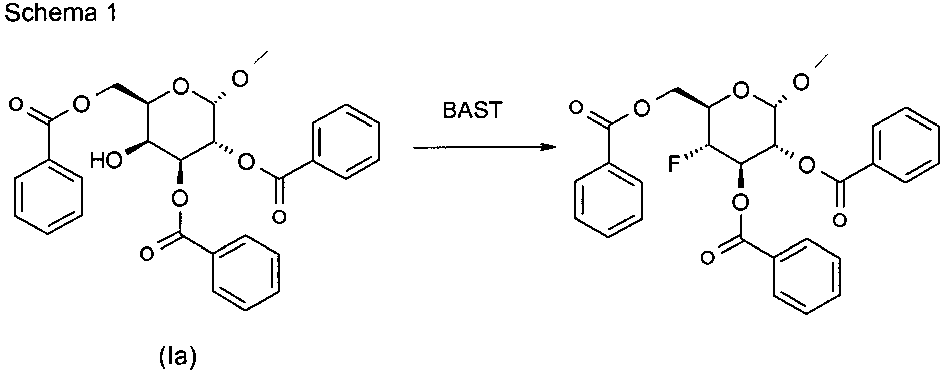

Das beschriebene Verfahren geht vom korrespondierenden Monoalkohol la (Reist et al. J. Org. Chem 1965, 30, 2312) aus und beinhaltend eine Kopplung mit Diethylaminoschwefeltrifluorid (DAST, Peter J. Card, J. Org. Chem. 1983, 48, 393) oder Bis(2-methoxyethyl)aminoschwefeltrifluorid (BAST). Diese Verfahren weisen jedoch bezogen auf eine technische Umsetzung verschiedene Nachteile auf. So werden teure und für die Umwelt problematische Fluorierungsmittel in großen Mengen benötigt.

Im Hinblick auf die vorstehend geschilderten Nachteile und Probleme besteht der Bedarf, ein Verfahren bereitzustellen, das diese Nachteile und Probleme vermeidet, das sich zudem, ohne großen zusätzlichen Aufwand zu erfordern, auf einfache Weise realisieren lässt und die gewünschten Produkte mit hohem Umsatz und hoher Selektivität in hohen Ausbeuten zugänglich macht, besonders für eine Produktion in großtechnischem Maßstab. Insbesondere hohe Ausbeuten und die Verwendung unproblematischerer Fluorierungsmittel sind eine zentrale Anforderung an das gesuchte Verfahren.

Diese Aufgabe wird gelöst durch ein Verfahren zur Herstellung von Verbindungen der allgemeinen Formel (I) 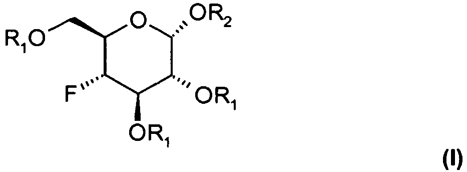 worin bedeuten
- R1: CO-R3;
- R2: (C₁-C₆)-Alkyl;
- R3: (C₁-C₆)-Alkyl, Phenyl, welches substituiert sein kann, bevorzugt unsubstituiertes Phenyl;
dadurch gekennzeichnet, dass

a) eine Komponente der Formel (II) 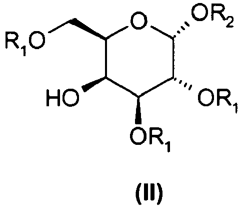 worin R1 und R2 wie oben definiert sind,
in einem geeigneten Lösungsmittel mit 0,1 bis 10 Äquivalenten, bevorzugt 0,8 bis 1,5 Äquivalenten eines Aktivierungsmittels, wobei das Aktivierungsmittel p-Nitrobenzolsulfonat (Nosylat) oder ein Fluorosulfonat ist; bevorzugt Fluorosulfonat, wie Triflat, Tresylat, Nonaflate, Triflat; besonders bevorzugt Triflat; in Gegenwart einer Base
bei - 50°C bis + 150 °C, bevorzugt bei - 20°C bis + 80 °C, besonders bevorzugt bei 0°C bis 25 °C umgesetzt wird zu der Verbindung (III), 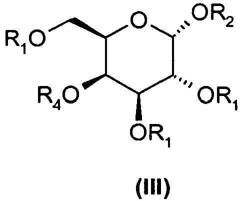 worin R1 und R2 die oben angegebenen Bedeutungen haben und
- R4: p-Nitrobenzolsulfonat (Nosylat) oder Fluorosulfonat bedeutet;
und anschließend

b) die Verbindung (III) mit 1 bis 10 Äquivalenten, bevorzugt 1,1 bis 2 Äquivalenten eines anorganischen Fluorierungsmittels, wie KF oder CsF, bevorzugt CsF in Gegenwart von katalytischen (5 - 10 mol%) Mengen eines Phasentransferkatalysators, bevorzugt Benzyltriethylammoniumfluorid, Tetraphenylphosphoniumfluorid, Tetrabutylammoniumfluorid, bevorzugt Tetrabutylammoniumfluorid in Gegenwart einer Säure in einem tertiären Alkohol bei - 50°C bis + 150 °C, bevorzugt bei - 20°C bis + 80 °C, besonders bevorzugt bei 0°C bis 25 °C umsetzt;
und gegebenenfalls anschließend die Verbindungen der Formel (I) durch gängige Reinigungsmethoden, wie Kristallisation, Destillation oder Chromatographie, bevorzugt durch Kristallisation aus einem oder einem Gemisch mehrerer Lösungsmittel, wie Alkanen, Aromaten, halogenierten Lösungsmitteln, Ether, Ketone, Ester, Alkoholen oder Wasser, reinigt, besonders bevorzugt durch Kristallisation aus Methanol oder aus Gemischen von Dichlormethan/Heptan bzw. Methanol/Wasser oder durch Reinigung über das Natrium-Salz und - nach Neutralisation - Kristallisation aus Wasser reinigt.

Geeignete Lösungsmittel im Verfahrensschritt a) sind beispielhaft übliche aprotische organische Lösungsmittel wie zum Beispiel Toluol, Chlorbenzol, Dichlormethan, Ethylacetat, Butylacetat, Diisobutylether, Diisopropylether, Tetrahydrofuran, 2-Methyltetrahydrofuran oder Methylethylketon, bevorzugt Toluol, Chlorbenzol, Dichlormethan oder Ethylacetat, besonders bevorzugt ist Dichlormethan oder Ethylacetat.
Geeignete Basen im Verfahrensschritt a) sind tertiäre Amine wie zum Beispiel Trialkylamine, wieTriethylamin, Ethyldimethylamin, Ethyldiisopropylamin, Tributylamin, oder N-Ethylmorpholin, Tetramethylethylendiamin oder Pyridin und substituierte Pyridine wie Lutidin oder Collidin, bevorzugt sind Triethylamin oder Pyridin.
Die Aktivierungsmittel Nosylat und Fluorosulfonate, wie Triflat, Tresylat, Nonaflate können sowohl als Sulfonylhalogenide als auch als Anhydride eingesetzt werden.

In Schritt a) wird die Komponente II in einem geeignetem Lösungsmittel in Gegenwart einer Base bei -30 °C bis + 40°C, vozugsweise bei -20°C bis +10°C, besonders bevorzugt bei -15°C bis 0°C gelöst und innerhalb von 15 -150 Minuten, vorzugsweise 20-40 Minuten zu einer auf -10°C bis 30°C, vorzugsweise -10°C bis 0°C gekühlten Lösung das Aktivierungsmittel, zudosiert.

Alternativ kann auch eine auf -30°C bis 40°C, vorzugsweise -20°C bis 10°C, besonders bevorzugt bei -15°C bis 0°C gekühlte Lösung des Aktivierungsmittels in einem geeigneten Lösungsmittel vorgelegt werden und dann die Komponente II in einem geeignetem Lösungsmittel in Gegenwart einer Base bei -30 °C bis + 40°C, vozugsweise bei -20°C bis +10°C, besonders bevorzugt bei -5°C bis 0°C innerhalb von 30 - 150 Minuten, vorzugsweise 60-120 Minuten zudosiert werden.

Das Reaktionsgemisch wird bei -10°C bis 40°C, bevorzugt bei -10°C bis 0°C 1 bis 150 Minuten gerührt, vorzugsweise 10 - 60 Minuten.

Das Reaktionsgemisch kann nun entweder direkt in die Folgereaktion eingesetzt werden, oder das gebildete Produkt der Formel III wird isoliert.

Zur Isolierung der Verbindung der Formel III wird das Lösungsmittel im Vakuum abgedampft. Optional wird das Reaktionsgemisch vor dem Abdampfen des Lösungsmittels mit Wasser gewaschen.

Geeignete Lösungsmittel im Verfahrensschritt b) sind tertäre Alkohole, wie beispielsweise t-Butanol, t-Amylalkohol,Tetrahydrolinalool, 3-Methyl-3-Pentanol, bevorzugt t-Butanol, t-Amylalkohol, besonders bevorzugt t-Amylalkohol.
Auch Mischungen der tertiären Alkohole mit üblichen aprotischen organischen Lösungsmitteln, wie zum Beispiel Toluol,Chlorbenzol, Dichlormethan, Ethylacetat, Butylacetat, Diisobutylether, Diisopropylether, Tetrahydrofuran, 2-Methyltertrahydrofuran, Dimethylformamid, N-Methylpyrrolidon sind möglich.

Geeignete Säuren im Verfahrensschritt b) sind beispielsweise eine oder mehrere Säuren - wobei eine Säure bevorzugt ist -, bevorzugt Lewis-Säuren, wie BF₃, FeCl₃, ZnGl₂, MgCl₂, ZnBr₂, MgBr₂, aber auch mit Brönsted-Säuren wie CF₃SO₃H, CH₃SO₃H, H₂SO₄, Toluolsulfonsäure, besonders bevorzugt mit CH₃SO₃H.

Die Alkylreste, einschließlich R1, R2 und R3 können sowohl geradkettig wie verzweigt sein.

Das erfindungsgemäße Verfahren zeichnet sich dadurch besonders aus, dass es in hohen Ausbeuten einen technisch durchführbaren Weg zu 4-Fluoro-4-desoxy- α-D-glucopyranosiden ermöglicht.

Die folgenden Bespiele erläutern das Verfahren, ohne es zu beschränken.

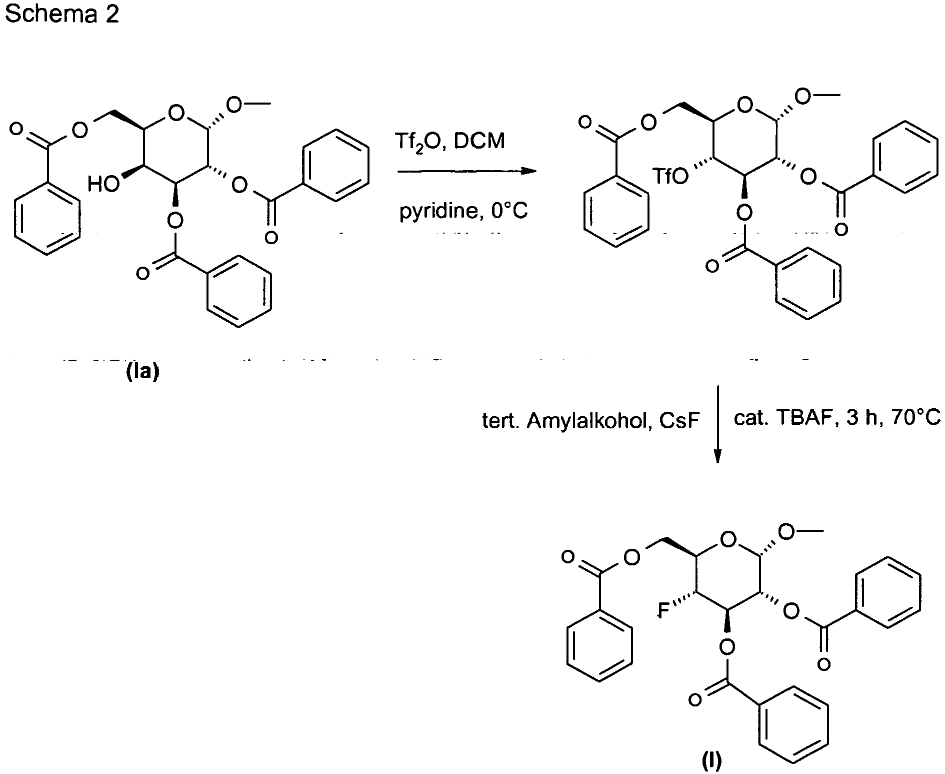

### Experimenteller Teil:

### Beispiel 1:

### Beispiel 1:

### Methyl 2,3,6-tri-O-benzoyl-4-O-trifluormethansulfonyl-a-D-glucopyranosid:

Zu einer gerührten Lösung von 50 g (99 mmol) Methyl 2,3,6-tri-O-benzoyl-4-hydroxy-α-D-glucopyranosid in 300 mL Methylenchlorid und 20 mL (2,5 eq.) Pyridin wurde 34 g (1,2 eq.) Trifluormethansulfonsäureanhydrid tropfenweise zugegeben bei -15°C - 0°C, über 30 Minuten. Zur Aufarbeitung wurde kaltes Wasser zugegeben und die entstehenden 2 Phasen getrennt. Die organische Phase wurde zweimal mit gesättigter NaHCO₃-Lösung, dann mit 1 N HCl und dann mit Wasser extrahiert. Nach Verdampfen des Lösungsmittels wurde ein gelber Feststoff erhalten. Ausbeute 94%.
¹H-NMR (d₆-DMSO): d = 3,47 (s, 3 H), 4,32 (dd 1 H, J = 6,2, 11,2 Hz), 4,4 (m, 1 H), 4,74 Ψt, 1 H, J = 6.6), 5,32 (d, 1 H, J = 3,6 Hz), 5,46 (m, 1 H), 5,99-6,03 (m, 2 H), 7,45-8,05 (m, 15 H) ppm.

### Beispiel 2

### Methyl 2,3,6-tri-O-benzoyl-4-fluoro-4-deoxy-a-D-glucopyranosid (I):

Zu einer gerührten Suspension von 2,5 eq. Cäsiumfluorid und einer katalytischen (5 - 10 mol%) Menge Tetrabutylammoniumfluorid und einer katalytischen Menge (10 mol%) Methansulfonsäure in tert. Amylalkohol wurde eine Lösung von Methyl 2,3,6-tri-O-benzoyl-4-O-trifluormethansulfonyl-α-D-glucopyranosid über 3 h bei 70°C zugegeben. Es wurde weitergerührt bis die HPLC-Kontrolle kein Ausgangsmaterial anzeigte. Die Reaktion wurde durch Zugabe von 2 eq. NaHCO₃ gestoppt und das Lösungsmittel im Vakuum entfernt. Der Rückstand wurde in Ethylacetat aufgenommen und die organische Phase wurde zweimal mit Wasser gewaschen. Nach Verdampfen des Lösungsmittels wurde der ölige Rückstand aus alkoholischer Lösung kristallisiert oder durch Chromatographie an Silicagel gereinigt. Es wurde ein farbloser Feststoff erhalten. Ausbeute >40%.
¹H-NMR (d₆-DMSO): d = 3,43 (s, 3 H), 4,31 (m 1 H), 4,56 (dd 1 H, J = 5,2, 12,2 Hz), 4,66 (m, 1 H), 5,10 (dΨt, 1 H, J = 9,4, 50,1 Hz), 5,20 (Ψt, 1 H, J = 3,4 Hz), 5,30 (dd 1 H, J = 3,4, 10,2 Hz), 5,90 (m, 1 H), 7,45-8,1 (m, 15 H) ppm.

## Patentansprüche

1. Verfahren zur Herstellung von Verbindungen der allgemeinen Formel (I): 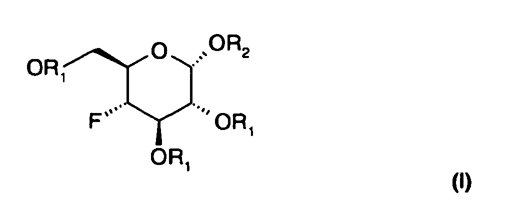 worin bedeuten
R1 CO-R3;
R2 (C₁-C₆)-Alkyl;
R3 (C₁-C₆)-Alkyl, Phenyl, welches substituiert sein kann;
**dadurch gekennzeichnet, dass**
a) eine Komponente der Formel (II) 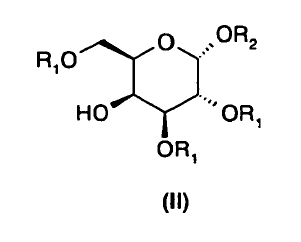 worin R1 und R2 wie oben definiert sind
in einem geeigneten Lösungsmittel mit 0,1 bis 10 Äquivalenten, bevorzugt 0,8 bis 1,5 Äquivalenten eines Aktivierungsmittels in Gegenwart einer Base, wobei das Aktivierungsmittel p-Nitrobenzolsulfonat (Nosylat) oder ein Fluorosulfonat ist,
bei - 50°C bis + 150 °C, umgesetzt wird zu der Verbindung (III); 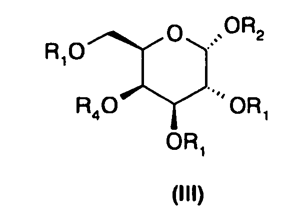 worin R1 und R2 die oben angegebenen Bedeutungen haben und
R4 p-Nitrobenzolsulfonat (Nosylat) oder Fluorosulfonat bedeutet;
und anschließend
b) die Verbindung (III) mit 1 bis 10 Äquivalenten, bevorzugt 1,1 bis 2 Äquivalenten eines anorganischen Fluorierungsmittels, in Gegenwart von katalytischen Mengen eines Phasentransferkatalysators, in Gegenwart einer Säure in einem tertiären Alkohol bei - 50°C bis + 150 °C
umsetzt.

2. Verfahren zur Herstellung von Verbindungen der allgemeinen Formel (I) nach Anspruch 1, **dadurch gekennzeichnet, dass** das Aktivierungsmittel Nosylat, Triflat, Tresylat oder Nonaflate ist.

3. Verfahren zur Herstellung von Verbindungen der allgemeinen Formel (I) nach Anspruch 1, **dadurch gekennzeichnet, dass** das Aktivierungsmittel Triflat ist.

4. Verfahren zur Herstellung von Verbindungen der allgemeinen Formel (I), nach Anspruch 1, 2 oder 3 **dadurch gekennzeichnet,**
**dass** das anorganische Fluorierungsmittel CsF ist.

## Claims

1. A process for preparing compounds of the formula (I) : 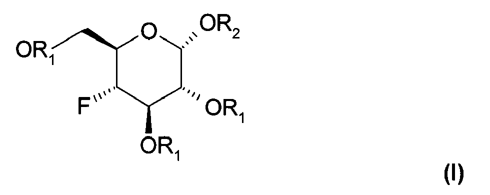 in which
R1 is CO-R3;
R2 is (C₁-C₆) -alkyl;
R3 is (C₁-C₆)-alkyl, phenyl, which may be substituted;
which comprises
a) converting a component of the formula (II) 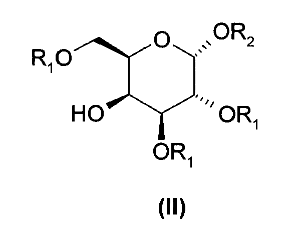 in which R1 and R2 are as defined above in a suitable solvent with 0.1 to 10 equivalents, preferably 0.8 to 1.5 equivalents, of an activating agent in the presence of a base, the activating agent being p-nitrobenzenesulfonate (nosylate) or a fluorosulfonate, at from -50°C to +150°C into the compound (III): 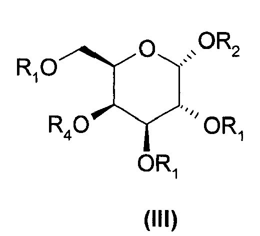 in which R1 and R2 have the meanings given above and
R4 is p-nitrobenzenesulfonate (nosylate) or fluorosulfonate;
and then
b) reacting the compound (III) with 1 to 10 equivalents, preferably 1.1 to 2 equivalents, of an inorganic fluorinating agent in the presence of catalytic amounts of a phase transfer catalyst, in the presence of an acid, at from -50°C to +150°C in a tertiary alcohol.

2. The process for preparing compounds of the formula (I) as claimed in claim 1, wherein the activating agent is nosylate, triflate, tresylate or nonaflate.

3. The process for preparing compounds of the formula (I) as claimed in claim 1, wherein the activating agent is triflate.

4. The process for preparing compounds of the formula (I) as claimed in claim 1, 2 or 3, wherein the inorganic fluorinating agent is CsF.

## Revendications

1. Procédé pour la préparation de composés de formule générale (I) : 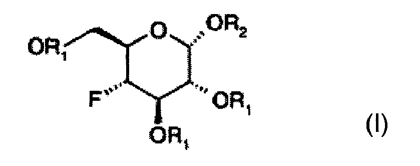 dans laquelle
R1 représente CO-R3 ;
R2 représente un groupe alkyle en C₁-₆;
R3 représente un groupe alkyle en C₁-C₆, phényle, qui peut être substitué ;
**caractérisé en ce que**
a) on convertit un composant de formule (II) 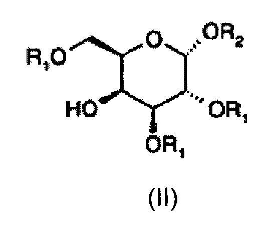 dans laquelle R1 et R2 sont tels que définis plus haut dans un solvant approprié, à l'aide de 0,1 à 10 équivalents, de préférence 0,8 à 1,5 équivalent d'un agent d'activation, en présence d'une base, l'agent d'activation étant le p-nitrobenzènesulfonate (nosylate) ou un fluorosulfonate, à une température de -50 °C à +150 °C, en le composé (III) : 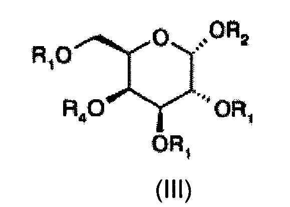 dans lequel R1 et R2 ont les significations indiquées plus haut et
R4 représente le p-nitrobenzènesulfonate (nosylate) ou le fluorosulfonate ;
et ensuite
b) on convertit le composé (III) à l'aide de 1 à 10 équivalents, de préférence 1,1 à 2 équivalents d'un agent de fluoration inorganique, en présence de quantités catalytiques d'un catalyseur de transfert de phase, en présence d'un acide, en un alcool tertiaire, à une température de -50 °C à +150 °C.

2. Procédé pour la préparation de composés de formule générale (I) selon la revendication 1, **caractérisé en ce que** l'agent d'activation est le nosylate, triflate, trésylate ou les nonaflates.

3. Procédé pour la préparation de composés de formule générale (I) selon la revendication 1, **caractérisé en ce que** l'agent d'activation est le triflate.

4. Procédé pour la préparation de composés de formule générale (I) selon la revendication 1, 2 ou 3, **caractérisé en ce que** l'agent de fluoration inorganique est le CsF.
